# EUROPEAN PATENT APPLICATION

(11) **EP 3 081 185 A1**
(43) Date of publication of application: **19.10.2016**
(21) Application number: 15003645.7
(22) Date of filing: 22.12.2015
(51) Int. Cl.: A61B 90/00, A61B 90/70

(54) **DEVICE AND METHOD FOR TREATMENT OF THROUGH-GOING HOLLOW SPACES OR LUMEN IN TOOLS**

(30) Priority: 22.12.2014 NL 1041124
(71) Applicant: Kemkers, Frederikus Anne, 9451 HT Rolde (NL)
(72) Inventor: Kemkers, Frederikus Anne, 9451 HT Rolde (NL)
(74) Representative: Ferguson, Alexander

(57) **Abstract**

Method for treating through-cavities or lumen of tools, in particular medical instruments, wherein the tool is inserted in a passage to a chamber defined by and within a housing wall, wherein the entrance end or proximal end of the tool is situated outside of the housing wall and the exit end or distal end of the tool is situated in the chamber, the tool is held in that position, compressed gas is introduced in the proximal end of the tool in order to press gas through the lumen from the proximal end towards the distal end of the tool while taking along particles present in the lumen.

## Description

### BACKGROUND OF THE INVENTION

The invention relates to a device for treating through-cavities or lumen of tools, in particular medical instruments. The invention furthermore relates to a method for treating through-cavities or lumen of tools, in particular medical instruments. The tools usually are elongated, wherein the through-cavities extend over at least almost the tool's length.

After use, hollow medical instruments, such as tubes for a scopic procedure, (bone) drills having an internal cooling channel and liposuction tools and curettes are washed in a Cleaning and Sterilization Department, CSD) in a washing machine and subsequently placed in racks or trays with other tools, geared to the next use, a specific operation. Just before the next use the racks or and/or trays in question are sterilized in a sterilization-device in the CSD in order to be used again. However, after the washing process, contaminating solid particles and moisture particles may be left behind in the lumen of the instrument. They constitute a health hazard, which grows even worse due to the period between washing and the sterilization process. The larger the size of the tool, the larger the problem will be.

After the washing process it occurs that, in view of drying, staff - without protection- blows through the instruments over a flannel cloth laid on a table or over a waste bin. However, that is not a reliable process. Furthermore, there is the risk of the contaminating particles ending up in the air and thus constituting a contamination hazard and as a consequence a health hazard, due to cross-contamination with other tools or due to persons getting infected.

It is furthermore known to make use of a permanently positioned suction device, provided with a recess, to which a suction line has been connected, leading to a central vacuum source in the institution in question. Staff holds an instrument to be treated, usually a bundle of instruments to be treated, in the recess by hand, such that the outer end(s) enter the sphere of influence of the vacuum. The treatment process is rather uncontrolled and unreliable, particularly when carried out with bundles of instruments.

There is a need for a device and method with which hollow medical instruments can be treated safely and reliably for removing contaminations left behind after washing.

### SUMMARY OF THE INVENTION

It is an object of the invention to provide a device and method with which tools having through-cavities or lumen, in particular medical instruments, can be treated safely and reliably for removing contaminations left behind after washing.

It is an object of the invention to provide a device with which tools having through-cavities or lumen, in particular medical instruments, can be treated for removing contaminations left behind after washing, which can be used by the staff with ease and overview.

It is an object of the invention to provide a method with which tools having through-cavities or lumen, in particular medical instruments, can be treated for removing contaminations left behind after washing, which can be carried out with ease, safely and with overview.

It is an object of the invention to provide a device with which tools having through-cavities or lumen, in particular medical instruments, can be treated for removing contaminations left behind after washing, which has a simple structure and/or is easy to clean.

It is an object of the invention to provide a device with which tools having through-cavities or lumen, in particular medical instruments, can be treated for removing contaminations left behind after washing, which is easy to handle and store.

For achieving at least one of those objects the invention according to one aspect provides a device for treating through-cavities or lumen of tools, in particular medical instruments, which treatment comprises having a gas flow through the lumen, wherein device comprises:
- a housing having a housing wall, the housing wall defining a chamber within there,
- wherein the housing wall has been provided with an entrance to the chamber and with an exit from the chamber,
- wherein the entrance forms a passage for the tool,
- wherein the entrance has been provided with a seal for sealing against gas flowing through the entrance passage along the exterior of the tool, and
- wherein the exit forms a passage for discharging gas from the chamber.

Such a device can be used with a tool, such as a compressed-air gun, with which pressurized gas, pressurized air, can be introduced into the end of the elongated tool situated outside the device. The gas flows forcefully through the lumen and takes along the (unwanted) particles present in there and exits from the end of the tool extending into the chamber. Apart from the entrance and exit, the chamber forms a space separated from the environment in a gas-tight manner, in which space the particles that have been taken along can at least be partially collected. The sealing prevents gas including particles from escaping to the outside along the exterior of the tool and end up in the environment unchecked, in particular on staff using the device. The discharge prevents too large an overpressure in the chamber that might hamper the flow-through process.

In one embodiment the entrance has been provided with holding means for -considered in lumen or longitudinal direction of the tool- keeping a tool extending through the passage in its place. The distal outer end of the tool can then remain reliably spaced apart from the housing wall. Staff can then pay more attention to the cleaning process and handling the compressed-gas gun or the like. This is also enhanced when the holding means are alternatively or also suitable for retaining the orientation of the tool then extending through the passage.

In one embodiment the seal comprises a first sealing member, for instance designed as a snap seal cap (shaft sealing) with a flexible flange. A return flow along the exterior of the tool is thereby prevented right from the start of introducing compressed gas. For preventing a return flow along the exterior of the tool at higher pressures, such as 1 to 2 bar overpressure, the device may have further been provided with a second sealing member that is in series with the first sealing member and is operational above a specific pressure difference, wherein the second sealing member is situated closer to the chamber than the first sealing member, wherein the second sealing member is a cross-slit valve, preferably a double cross-slit valve. In an inoperative condition, the second sealing member can ensure sealing to prevent migration of dirt particles through the passage to the outside.

In an advantageous arrangement the first and second sealing members are part of said holding means.

To enhance keeping the chamber clean itself, the housing wall can have an inner surface that is at least partially smoothly curved concave, in particular have an inner surface that at least in one plane of cross-section of the housing wall is smoothly curved concave over the circumference. Preferably the housing wall has an inner surface that is at least almost entirely smoothly curved concave.

In one embodiment, in which depositing particles taken along from the lumen in angles that are relatively dead spots for the flow, is counteracted as much as possible, the housing wall forms at least almost a three-dimensional oval, preferably at least almost an ellipsoid. Alternatively, the housing wall can at least almost define a sphere. This largely prevents air flowing out of the tool from bouncing back, and enhances the distribution of the air in the chamber.

To enhance keeping the chamber clean, the housing wall can be composed of parts, which parts can at least partially be removed from each other, and can be connected to each other in a gas-sealing manner. In an easy to handle embodiment, the housing wall is two-part, preferably composed of two halves, preferably according to a division in a horizontal plane. It may for instance regard parts that can be fully detached from one another or parts that are hinged to each other.

In one embodiment in which the discontinuities are kept limited to a large extent, and which may also be advantageous in terms of manufacturing, the entrance and the exit have been arranged in the same housing wall part, preferably in a same mounting part. It is ergonomically advantageous if that is the case in an upper housing wall part.

In view of the removability and sealing during use, the device may have been provided with clamps for clamping the parts of the housing wall to each other.

For keeping it in its place, the device according to the invention may comprise a frame for supporting the housing wall on a basis, preferably only by free support. This will result in more freedom of designing the shape of the housing wall. The device can easily be moved. The frame may comprise columns that may or may not be connected to each other by other parts of the frame.

In one embodiment that is advantageous in terms of structure, the columns support the clamps, preferably at the upper end of the columns.

The frame is able to engage the housing wall in a supporting manner near the division of the housing wall. In the above-mentioned horizontal division in two of the housing wall, the frame can engage a lower half of the housing wall in a supporting manner, wherein the clamps comprise clamping members which extend from the supporting frame in order to engage the upper half of the housing wall and clamp it to the lower half. The clamping members can then be adjusted between a position releasing the upper half of the housing wall (for taking out and disassembling the housing) and a position clamping the upper half to the lower half of the housing wall.

In a further development the exit has been provided with a discharge line for discharging gas/air from the chamber to a location of discharge, so that the discharge thereof is controlled. In order to prevent any particles that have not been deposited in the chamber from nonetheless ending up in the environment, the device may have been provided with an air filter (HEPA quality) having an entrance connected to the discharge line and having an exit for filtered air. In view of easily cleaning the filter, the discharge line and the entrance of the air filter can be detachably connected to each other. Its cleaning is facilitated if the air filter comprises a replaceable filter element.

In a compact embodiment the air filter is situated below the housing. It is advantageous when said frame carries both the housing and the air filter.

If the air filter is situated below the housing, the discharge line can be kept short if the exit is situated in a lower part of the housing wall. In the above-mentioned case of a housing wall divided horizontally in two, the entrance can then be situated in the upper part and the exit in the lower part. Both parts can be shaped identically so that one moulding tool would suffice.

In order to be able to follow where the distal end of the tool is situated within the chamber and to make it possible to establish at an early stage, contaminations that may be dangerous to the staff (inspection), the housing wall may at least partially have been made of at least translucent material, preferably of transparent material. In case of said division into two, the upper half may have been made of at least translucent material, preferably of transparent material, so that staff is able to keep optimal track of what goes on. The housing wall may be almost entirely translucent, preferably transparent.

The device according to the invention can be compact and easy to move by staff, from a location of storage to the location of use and back again. Storage requires little room. The device can be easily disassembled and assembled again. The parts of the device can be cleaned easily and thoroughly.

According to a further aspect, the invention provides a method for treating through-cavities or lumen of tools, in particular medical instruments, wherein the tool, which is in particular elongated, is inserted in a passage to a chamber defined by and within a housing wall, wherein the entrance end or proximal end of the tool is situated outside of the housing wall and the exit end or distal end of the tool is situated in the chamber, the tool is held in that position, pressurized gas is introduced into the proximal end of the tool in order to press gas through the lumen from the proximal end towards the distal end of the tool while taking along particles present in the lumen.

Preferably during introducing the compressed gas the escape of gas from the chamber according to a path along the circumference of the tool is obstructed.

The gas introduced into the chamber preferably is given the opportunity to escape via a discharge line and preferably is filtered while doing so, in order to safeguard the environment reliably from particles removed from the lumen.

The controllability of the process by staff is enhanced if the tool is held stably in its place and oriented in the passage. The tool can then be free from contact with the inner surface of the housing wall.

Specifically, in the method according to the invention use can be made of a device according to the invention.

The aspects and measures described in this description and the claims of the application and/or shown in the drawings of this application may where possible also be used individually. Said individual aspects may be the subject of divisional patent applications relating thereto. This particularly applies to the measures and aspects that are described per se in the sub claims.

### BRIEF DESCRIPTION OF THE DRAWINGS

The invention will be elucidated on the basis of an exemplary embodiment shown in the attached drawings, in which:
Figure 1 shows a view of an exemplary embodiment of a device according to the invention;
Figures 2A-D show some details of the device of figure 1, the entrance in exploded condition, a cross-section of a mounting part for the entrance and exit, a cross-section of a filter of the device of figure 1 and a cross-section of a support of the device of figure 1, respectively; and
Figures 3A and 3B show the device of figure 1 in operation.

### DETAILED DESCRIPTION OF THE DRAWINGS

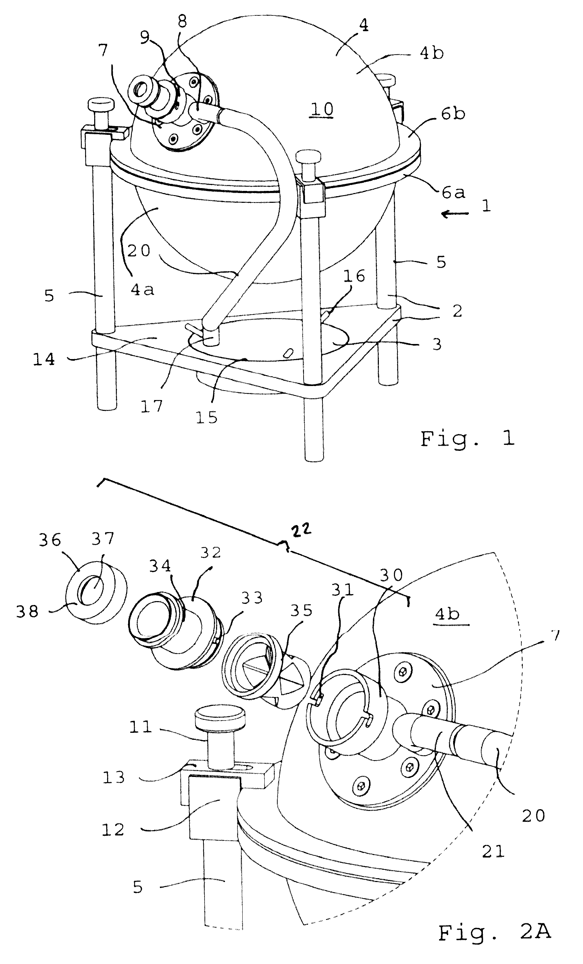

The device 1 of figure 1 comprises a frame 2 in which a collection housing 4 and a filter housing 3 are supported.

The collection housing 4 defines a spherical chamber 10 composed of two sphere-halves 4a,4b. The lower sphere-half 4a, which can be made of stainless steel, is entirely closed, the upper sphere-half 4b, which may for instance be made of polycarbonate (Lexan) or PPSU, has been provided with a stainless steel mounting part 7 in its wall, to which mounting part two air passages 8 and 9 towards and from the chamber 10 have been provided. The lower sphere-half may alternatively have been made of the same material as the upper sphere-half, and then for instance also be transparent.

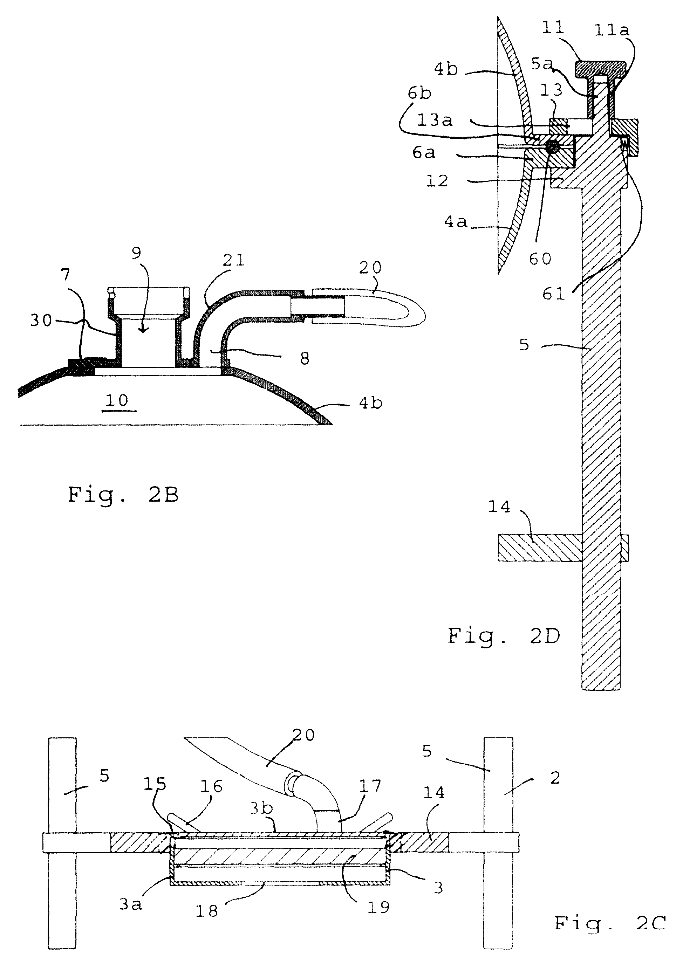

As can also be seen in figure 2A, at the location of the passage 8 an elbow pipe 21 has been attached, to which a discharge hose 20, for instance made of silicone material, has been connected (figures 2A,B).

At the location of the passage 9, a stainless steel instrument holder 22 has been arranged, which (figure 2A) comprises a first spout-shaped mounting part 30 including bayonet collar 31. A so-called double silicone valve 35, also called a double pyramid valve or double cross-slit valve, fits in the collar 31. Said valve has been attached to a second mounting part 32 of the instrument holder 22, that has been provided with bayonet pins and with a flange 33, forming one unity with a spout 34. A sealing 36, also called snap seal cap, provided with an opening 37 surrounded by a flange 38, fits on the spout 34.

The sphere-halves 4a,4b have been provided with circumferential flanges 6a,b with which they have detachably been clamped onto each other, with a silicone sealing ring 60 in between them (figure 2D). The clamping is effected here by two clamps 12 and 13, wherein clamp 12 each time is integrally formed with the column 5 in question. The lower clamp 12 forms a bearing surface for the flange 6a of the lower part 4a, and the upper clamp 13 abuts the upper side of the flange 6b of the upper part 4b. A rotary knob 11 with internally threaded bore hole 11 a has been screwed onto a threaded end 5a and engages onto the top surface of upper clamp 13. The threaded end 5a extends through a horizontal elongated hole 13a in clamp 13. A spring 61 biasses the clamp 13 radially to the outside, so that it is shifted with the elongated hole 13a in horizontal, outward direction along the threaded end 5a as soon as the rotary knob 11 releases the clamp 13, and the flange 6b is released. Alternatively, it is also possible to let the threaded end be one unity with the rotary knob 11 and then have it engage into an internally threaded vertical bore hole in the column 5. By means of a rotary knob 11 the clamps 12 and 13 can be moved towards and away from each other. The rotary knob 11 can be loosened entirely.

Straight below the housing 4, the columns 5 support a plate 14, which (figure 2C) has been provided with a hole 15, through which the filter housing 3 extends. The filter housing has been formed more or less straight circle cylindrical, and with pins 16 supports on the edge of the hole 15. The filter housing 3 is also two-part, having a lower part 3a and an upper part 3b, that are detachably connected to each other. In the upper wall of the upper part 3b a spout 17 has been provided, to which the hose 20 can be connected. The lower wall of the lower part 3a has been provided with an air outlet opening 18, freely debouching, spaced apart from a basis. In the filter housing 3 a replaceable medical filter 19 (HEPA quality) has been placed, in the path between the spout 17 and the opening 18.

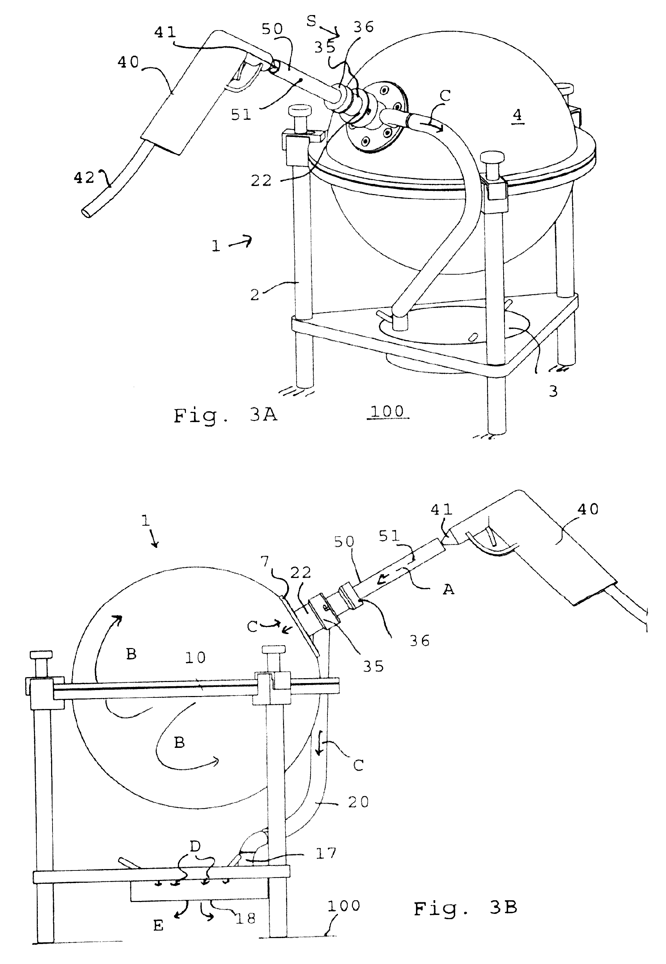

In operation, figures 3A and 3B, the device 1 has been placed on a table 100 by a member of staff. Then a rigid, elongated, tubular instrument 50 having lumen 51, that has just been washed in a washing machine, is inserted into the instrument holder 22, direction S, through the snap seal cap 36, the valve 35, until the (distal) outer end of the instrument 50 has largely ended up in the chamber 10, yet spaced apart from the housing wall, which can easily be followed and seen through the transparent sphere-half 4b. Both closures 36 (first sealing member) and 35 (second sealing member) constitute holding means that retain the tube 50 in longitudinal direction against shifting and keep it more or less in the orientation of insertion. Subsequently the barrel 41 of compressed air gun 40 is placed in the proximal outer end of the tube 50. The gun 40 has been connected to a compressed air hose 42, that has been connected to a central compressed air system, at an air pressure of for instance 1 to 2 bar overpressure.

As can be seen in figure 3B, compressed air forcefully flows through the lumen 51 of tube 50, direction A, which air flow drags moisture and other particles along. The air flowing out, exits from the distal outer end of the tube 50 and hits the sphere wall, and deflects in directions B. The air in the chamber 10 cannot escape along the outer circumference of the tube 50, as at a first stage of building up the air pressure in the chamber 10, the flange 37 of seal cap 36 abuts the tube 50 in a gas-sealing manner, and at a higher pressure the valve 35 ensures the sealing along the tube 50 (due to the slits said valve will not be active until after some pressure has been built up). Both sealings 36 and 35 complete each other, so that sealing in applied pressures is guaranteed.

As a consequence, air can only escape in direction C through the elbow pipe 21 and through the hose 20, towards the filter housing 3. At that location the air has to pass through the air filter 19, direction D, for filtering, and then exits, direction E, to the environment through opening 18.

The particles blown out of the tube 50 have been collected in the chamber 10 or intercepted in air filter 19. Fine particulate matter will have been removed from the air flow E exiting from the opening 18. The air exiting into the environment of the device 1 will be sufficiently clean and does not constitute a safety or contamination hazard.

The tube, that now has no undesired particles in the lumen, will then be suitable for sterilization in another dedicated device in the cleaning and sterilization department (CSD) of a hospital, without any contamination hazard.

The snap seal cap 36 can easily be replaced from the outside. The valve 35 can be replaced by loosening mounting part 32 from mounting part 30. This can for instance be done on a daily basis. The collection chamber 4 can also be cleansed and sterilized on a daily basis, to which end the clamps 12 and 13 are released and first the upper half 4b and then the lower half 4a are removed from the frame 2. The hose 20 can for instance be replaced on a weekly basis, just like air filter 19. The valve 35 ensures sealing of the passage when no tool extends through the passage.

The device 1 is limited in size (sphere cross-section is for instance 0.2 to 0.3 m) and can easily be moved by a member of staff to another location of use or to a storage. With its legs, the device 1 can be placed stably on a table at an ergonomically advantageous level.

The device according to the invention comprises relatively few parts. The entire device is detachable to a large extent and can be cleaned properly by washing in a washing machine and by subsequent sterilization in an autoclave. The device according to the invention is simple, without storage of treatment fluids, fluid pumps, sprinklers etcetera and can be lightweight.

The housing 2 of the device 1 of figure 1 is an example of a device according to the invention. Other embodiments are possible, such as an embodiment in which the sphere has been replaced by a cylinder having an upper wall and a lower wall. The cylinder preferably has a curved circumference, such as according to an ellipse or circle. The upper wall and the lower wall may be flat, for ease of manufacturing, or be convex, in which case also the formation of areas onto which dirt can easily deposit can be counteracted.

In one divided embodiment of the housing, such as in two parts, the housing parts can be hinged to each other, and then for instance in the closed position be clamped to each other using any suitable means. Housing parts can alternatively also be detachably placed on each other in a different way, for instance using a bayonet catch.

The above description is included to illustrate the operation of preferred embodiments of the invention and not to limit the scope of the invention. Starting from the above explanation many variations that fall within the spirit and scope of the present invention will be evident to an expert. Equivalents of the parts to be used of the device according to the invention also fall within the scope of the invention.

## Claims

1. Device for treating through-cavities or lumen of tools, in particular medical instruments, which treatment comprises having a gas flow through the lumen, wherein the device comprises:
- a housing having a housing wall, the housing wall defining a chamber within there,
- wherein the housing wall has been provided with an entrance to the chamber and with an exit from the chamber,
- wherein the entrance forms a passage for the tool,
- wherein the entrance has been provided with a seal for sealing against gas flowing through the entrance passage along the exterior of the tool, and
- wherein the exit forms a passage for discharging gas from the chamber.

2. Device according to claim 1, wherein the entrance has been provided with holding means for -considered in longitudinal direction of the tool- keeping a tool extending through the passage in its place and/or with holding means for retaining the tool's orientation.

3. Device according to claim 1 or 2, wherein the seal comprises a first sealing member, designed as a snap seal cap (shaft sealing) with a flexible flange, wherein the seal preferably has furthermore been provided with a second sealing member that is in series with the first sealing member and is operational above a specific pressure difference, wherein the second sealing member is situated closer to the chamber than the first sealing member, wherein the second sealing member is a cross-slit valve, preferably a double cross-slit valve.

4. Device according to claim 2 and according to claim 3, wherein the first and second sealing members are part of the holding means.

5. Device according to any one of the preceding claims, wherein the housing wall has an inner surface that is at least partially smoothly curved concave, wherein, preferably, the housing wall has an inner surface that at least in one plane of cross-section of the housing wall is smoothly curved concave over the circumference, and/or wherein, preferably, the housing wall has an inner surface that is at least almost entirely smoothly curved concave, and/or wherein the housing wall forms at least almost a three-dimensional oval, preferably at least almost an ellipsoid, or at least almost defines a sphere.

6. Device according to any one of the preceding claims, wherein the housing wall has been composed of parts, which parts can at least partially be removed from each other, in particular are detachable from each other, and can be connected to each other in a sealing manner, preferably can be clamped to each other by clamps, wherein, preferably, the housing wall is two-part, preferably composed of two halves, preferably according to a division in a horizontal plane, wherein, preferably, the entrance and the exit have been arranged in the same housing wall part, preferably in the same mounting part and/or preferably in an upper housing wall part.

7. Device according to any one of the preceding claims, comprising a frame for supporting the housing wall, preferably only by free support, wherein, preferably, the frame comprises columns that may or may not be connected to each other by other parts of the frame, wherein, in case of the housing wall composed of parts according to claim 6, the frame preferably engages the housing wall near the division of the housing wall, in a supporting manner.

8. Device according to claim 6 and according to claim 7, wherein the housing wall has been composed of parts, which parts can at least partially be removed from each other, in particular are detachable from each other, and can be connected to each other in a sealing manner, can be clamped to each other by clamps, wherein the said columns support the clamps, preferably at the upper end of the columns.

9. Device according to claim 7 or 8, wherein the housing wall is two-part, preferably composed of two halves, preferably according to a division in a horizontal plane, wherein the frame engages a lower half of the housing wall in a supporting manner and the clamps comprise clamping members which extend from the frame in order to engage the upper half of the housing wall and clamp it to the lower half, wherein, preferably, the clamping members can be adjusted between a position releasing the upper half of the housing wall and a position clamping the upper half to the lower half of the housing wall.

10. Device according to any one of the preceding claims, wherein the exit has been provided with a discharge line for discharging gas/air from the chamber to a location of discharge, preferably an air filter (preferably HEPA quality) having an entrance connected to the discharge line and having an exit for filtered air, wherein, preferably, the discharge line and the entrance of the air filter can be detachably connected to each other and/or, wherein, preferably, the air filter comprises a replaceable filter element,
and/or wherein, preferably the air filter is situated below the housing,
and/or wherein, in case of said frame, the frame carries both the housing and the air filter.

11. Device according to any one of the preceding claims, wherein the housing wall has at least partially been made of at least translucent material, preferably of transparent material, wherein, in case the housing wall is two-part, preferably composed of two halves, preferably according to a division in a horizontal plane, the upper half has preferably been made of at least translucent material, preferably of transparent material.

12. Method for treating through-cavities or lumen of tools, in particular medical instruments, wherein preferably use is made of a device according to any one of the preceding claims, wherein the tool, that is in particular elongated, is inserted in a passage to a chamber defined by and within a housing wall, wherein the entrance end or proximal end of the tool is situated outside of the housing wall and the exit end or distal end of the tool is situated in the chamber, the tool is held in that position, compressed gas, preferably using a hand-held compressed gas gun or the like, preferably at a pressure of 1 to 2 bar overpressure, is introduced in the proximal end of the tool in order to press gas through the lumen from the proximal end towards the distal end of the tool while taking along particles present in the lumen.

13. Method according to claim 12, wherein during introducing compressed gas the escape of gas from the chamber according to a path along the circumference of the tool is obstructed and/or wherein the gas introduced into the chamber is given the opportunity to escape via a discharge line and preferably is filtered while doing so.

14. Method according to claim 12 or 13, wherein the tool is held stably in its place and oriented in the passage, preferably with the distal outer end at a distance from the inner surface of the housing wall.

15. Method according to any one of the claims 12, 13 or 14, wherein the step of pressing the compressed gas through the lumen is carried out after a washing treatment of the tool and prior to it being sterilized.

16. Device provided with one or more of the characterising measures described in the attached description and/or shown in the attached drawings.

17. Method provided with one or more of the characterising measures described in the attached description and/or shown in the attached drawings.
